Europäisches Patentamt

European Patent Office

Office européen des brevets

(19)

(11) EP 1 627 599 A2

(12) EUROPEAN PATENT APPLICATION

(43) Date of publication:
22.02.2006 Bulletin 2006/08

(51) Int Cl.:
A61B 5/107 (2006.01)    A61B 5/00 (2006.01)

(21) Application number: 05017897.9

(22) Date of filing: 17.08.2005

(84) Designated Contracting States:
AT BE BG CH CY CZ DE DK EE ES FI FR GB GR
HU IE IS IT LI LT LU LV MC NL PL PT RO SE SI
SK TR
Designated Extension States:
AL BA HR MK YU

(30) Priority: 20.08.2004 JP 2004241500

(71) Applicant: MATSUSHITA ELECTRIC INDUSTRIAL
CO., LTD.
Kadoma-shi, Osaka 571-8501 (JP)

(72) Inventors:
• Kondoh, Kazuya
  Katano-shi
  Osaka 576-0022 (JP)
• Uchida, Shinji
  Neyagawa-shi
  Osaka 572-0807 (JP)

(74) Representative: Grünecker, Kinkeldey,
Stockmair & Schwanhäusser
Anwaltssozietät
Maximilianstrasse 58
80538 München (DE)

(54) **Biological information arithmetic apparatus, biological information arithmetic method, program, and recording medium**

(57) An optical fat thickness meter of the present invention includes a light source section which is provided on a measuring surface and which emits light to a living body, a photoreception section which is provided on the measuring surface, and which receives the light passing the living body, a protruding section which is provided so as to surround the light source section and photoreception section substantially, and an arithmetic section which calculates information with regard to a living body on the basis of the amount of the received light.

Fig.1

**Description**

**BACKGROUND OF THE INVENTION**

**Technical field of the invention**

The present invention relates to a biological information arithmetic apparatus, a biological information arithmetic method, a program, and a recording medium for measuring partial subcutaneous fat thickness and the like using, for example, light.

**Related Art of the invention**

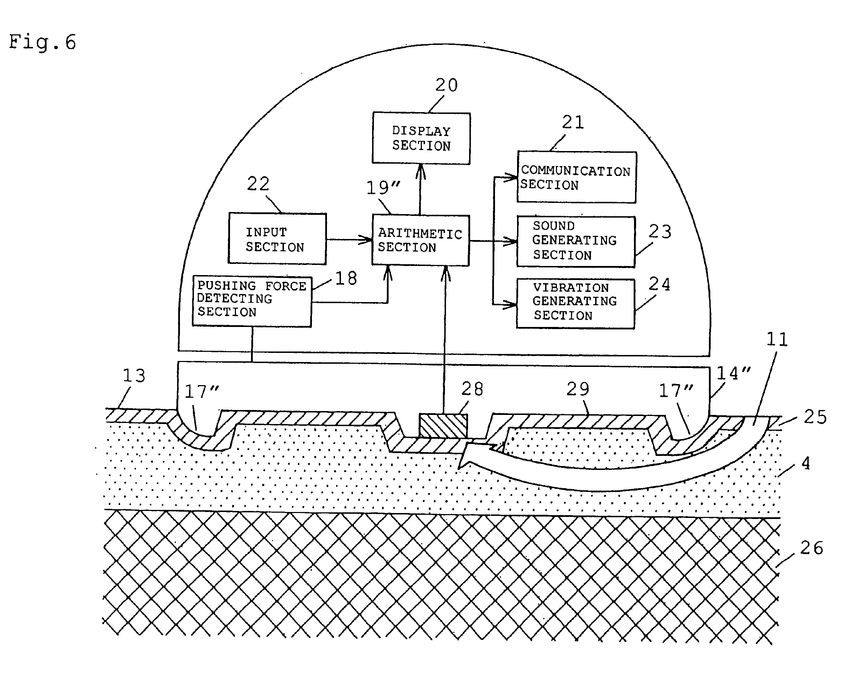

As conventional optical biological information measuring apparatuses whichmeasure partial subcutaneous fat thickness or the like, there have existed apparatuses which have measured the thickness of fat with diffuse reflection by locating a light source and a photoreception section on a living body surface (see Japanese Patent Laid-Open No. 11-239573 (e.g., page 4, Figure 6) or the like) .

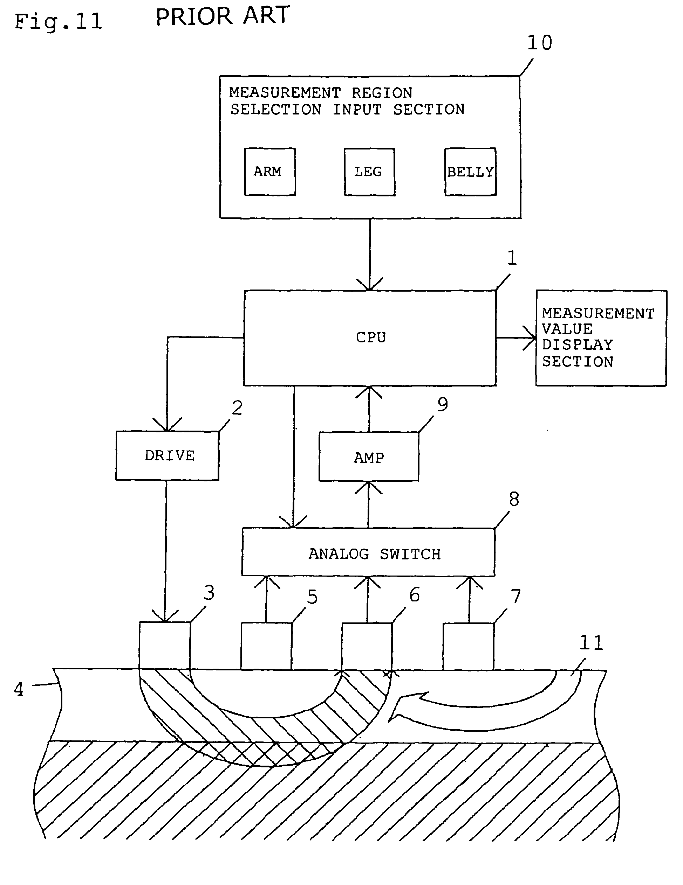

Figure 11 is a schematic sectional view of such a conventional optical fat thickness meter.

A drive circuit 2 drives a light transmitting device 3 by a command from a CPU 1, and the light transmitting device 3 driven emits near-infrared light.

This near-infrared light is received by any one of light receiving devices 5, 6, and 7 corresponding to an arm, a leg, and a belly, which are selected with an analog switch 8, while being given scattering absorption through subcutaneous fat 4, and is fetched into the CPU 1 through an AMP 9.

Here, the CPU 1 determines whether to use which output of the light receiving devices 5, 6, and 7 according to which of buttons is selected in a measurement region selective input section 10, and it makes the analog switch 8 operate.

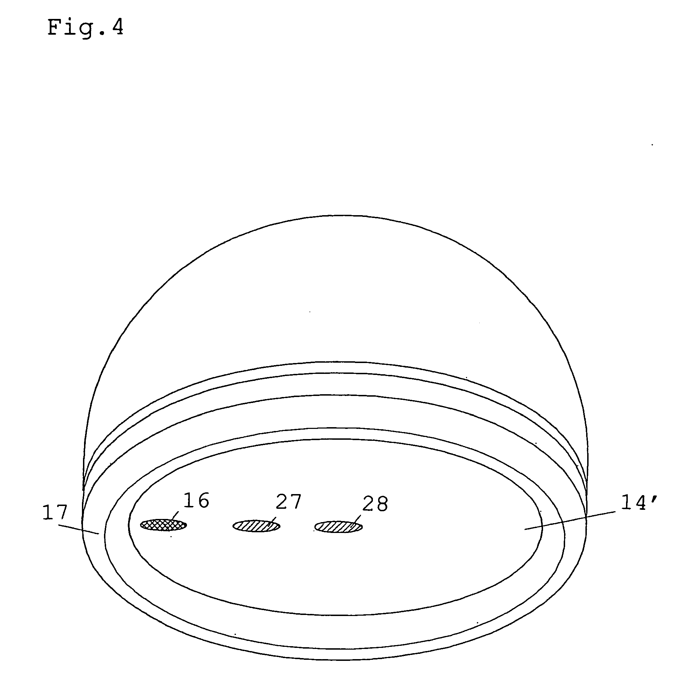

In addition, as other conventional art, there existed some which located a light transmitting device and a light receiving device so that several different optical path lengths may be further obtained, and perform the dispersion correction of color of a skin and the like from the amount of received light, which is obtained (see Japanese Patent Laid-Open No. 2000-155091 (e.g., page 5, Figure 4) or the like).

By the way, as for the optical fat thickness meters in the conventional art mentioned above, experiment ones have been mainly produced up to now, and hence, usually, they are used in environments, where unnecessary light is few, such as an interior of a laboratory.

Nevertheless, the present inventor recognized that light receiving devices 5, 6, and 7 receive not only light from the light transmitting device 3 but also disturbance light 11 which is sunlight or illumination light from lighting equipment which comes through a living body after illuminating a living body surface when such an optical fat thickness meter is used in an environment where unnecessary light is much (refer to Figure 11).

Then, the present inventor discerned that, since the disturbance light 11 was received, an S/N ratio (signal-to-noise ratio) became small, and hence, there existed a possibility that the accuracy of measurement of partial subcutaneous fat thickness and the like might deteriorate in an optical fat thickness meter at a practical use level.

In addition, the tendency for such an optical fat thickness meter to aim at the miniaturization of a device towards utilization has become remarkable.

Nevertheless, the present inventor found that, in such an optical fat thickness meter, the size of a measuring surface including the light transmitting device 3 and light receiving devices 5, 6, and 7 also became small as the miniaturization of a device advances (refer to Figure 11).

Then, the present inventor perceived that, when the influence of the disturbance light received became large in connection with the size of the measuring surface becoming small, the S/N ratio became small, and hence, there existed a possibility that the accuracy of measurement might deteriorate.

The present invention takes such conventional subjects mentioned above into consideration to aim at providing a biological information arithmetic apparatus, a biological information arithmetic method, a program, and a recording medium which can further enhance the accuracy of measurement of partial subcutaneous fat thickness and the like.

**SUMMARY OF THE INVENTION**

The 1st aspect of the present invention is a biological information arithmetic apparatus, comprising:

a light source emitting light to a living body, which is provided on a measuring surface;
photodetector receiving the light having passed through the living body, which is provided on the measuring surface;
a protruding section which is provided so as to substantially surround the light source and the photodetector; and
a biological information arithmetic unit of calculating information with regard to the living body on the basis of an amount of the received light.

The 2nd aspect of the present invention is the biological information arithmetic apparatus according to the 1st aspect of the present invention, wherein the photodetector receives the light also in a state where the light is not emitted while receiving the light in a state where the light is emitted, and the biological information arithmetic unit calculates the information with regard to the living body on the basis of the amount of the light received in the state where the light is not emitted, and the amount of the light received in the state where the light is emitted.

The 3rd aspect of the present invention is the biological information arithmetic apparatus according to the 1st aspect of the present invention, wherein the protruding section is continuously provided along an outer periphery of the measuring surface.

The 4th aspect of the present invention is the biological information arithmetic apparatus according to the 3rd aspect of the present invention,
wherein the outer periphery of the measuring surface is substantially a circumference, and
the photodetector is located in a substantial center of the circumference.

The 5th aspect of the present invention is the biological information arithmetic apparatus according to the 1st aspect of the present invention, further comprising a convex section provided so as to bridge in the protruding section,
wherein the light source is located in the convex section, and
the photodetector is located in the convex section.

The 6th aspect of the present invention is the biological information arithmetic apparatus according to the 5th aspect of the present invention,
wherein the outer periphery of the measuring surface is substantially a circumference, and
the convex section is provided so as to cross a substantial center of the circumference.

The 7th aspect of the present invention is the biological information arithmetic apparatus according to the 5th aspect of the present invention, further comprising a convex section pushing force measuring unit of measuring a pushing force by which the convex section is pushed on a surface of the living body,
wherein the biological information arithmetic unit calculates the information with regard to the living body in consideration of a deformation of the surface of the living body which is caused by the measured pushing force.

The 8th aspect of the present invention is the biological information arithmetic apparatus according to the 1st aspect of the present invention,
wherein the photodetector has a first light receiving device that receives the light, and a second light receiving device that receives the light, and
the biological information arithmetic unit calculates the information with regard to the living body on the basis of an amount of the light received by the first light receiving device, and an amount of the light received by the second light receiving device.

The 9th aspect of the present invention is the biological information arithmetic apparatus according to the 8th aspect of the present invention,
wherein the light source has a light emitting device which emits the light,
a distance between the light emitting device and the first light receiving device is substantially from 15 mm or more to 25 mm or less, and
a distance between the light emitting device and the second light receiving device is substantially from 35 mm or more to 50mm or less.

The 10th aspect of the present invention is the biological information arithmetic apparatus according to the 1st aspect of the present invention, further comprising:

 a measuring surface pushing force measuring unit of measuring a pushing force by which the measuring surface is pushed on a surface of the living body; and
 a judging unit of judging whether a magnitude of the measured pushing force is over a predetermined value,

wherein the biological information arithmetic unit calculates the information with regard to the living body if the magnitude of the measured pushing force is judged to be over the predetermined value.

The 11th aspect of the present invention is a biologicalinformation arithmetic method,comprising the steps of:

 emitting light using a light source which is provided on a measuring surface so as to be substantially enclosed by a protruding section and which emits light to a living body;
 performing photoreception using a photodetector which is provided on the measuring surface so as to be substantially enclosed by the protruding section and which receives light having passed through the living body; and
 performing an arithmetical operation using a biological information calculator which calculates information with regard to the living body on the basis of an amount of the received light.

The 12th aspect of the present invention is a program for making a computer execute the step of performing an arithmetical operation using a biological information calculator which calculates information with regard to the living body on the basis of an amount of the received light, in the biological information arithmetic method according to the 11th aspect of the present invention.

The 13th aspect of the present invention is a recording medium recording the program according to the 12th aspect of the present invention, which can be processed by a computer.

The present invention has an advantage of being able to further enhance the accuracy of measurement of partial subcutaneous fat thickness and the like.

**BRIEF DESCRIPTION OF THE DRAWINGS**

 Figure 1 is a schematic sectional view of an optical fat thickness meter of a first embodiment of the present invention.

Figure 2 is an outside view of the optical fat thickness meter of the first embodiment of the present invention.

Figure 3 is a schematic sectional view of an optical fat thickness meter of a second embodiment of the present invention.

Figure 4 is an outside view of the optical fat thickness meter of the second embodiment of the present invention.

Figure 5 is a schematic sectional view of an optical fat thickness meter of a third embodiment of the present invention (part 1).

Figure 6 is a schematic sectional view of an optical fat thickness meter of a third embodiment of the present invention (part 2).

Figure 7 is an outside view of the optical fat thickness meter of the third embodiment of the present invention.

Figure 8 is a schematic partially-sectional view of an optical fat thickness meter with a protruding section 117 in an embodiment of the present invention.

Figure 9 is a schematic bottom view of an optical fat thickness meter with a protruding section 217 according to an embodiment of the present invention.

Figure 10 is a schematic bottom view of an optical fat thickness meter with protruding sections 317a, 317b, 317c, and 317d according to an embodiment of the present invention.

Figure 11 is a schematic sectional view of a conventional optical fat thickness meter.

**Description of Symbols**

| 1 | CPU |
|---|---|
| 2 | Drive Circuit |
| 3 | Light transmitting device |
| 4 | Subcutaneous Fat |
| 5 | Light receiving device |
| 6 | Light receiving device |
| 7 | Light receiving device |
| 8 | Analog Switch |
| 9 | AMP |
| 10 | Measurement Region Selective Input Section |
| 11 | Disturbance Light |
| 13 | Living Body Surface |
| 14, 14', 14" | Measuring Planes |
| 15 | Photoreception section |
| 16 | Light Source Section |
| 17 and 17" | Protruding Sections |
| 18 | Pushing Force Detecting section |
| 19, 19', 19" | Arithmetic Sections |
| 20 | Display Section |
| 21 | Communication section |
| 22 | Input Section |
| 23 | Sound Generating Section |
| 24 | Vibration Generating Section |
| 25 | Skin |
| 26 | Muscle |
| 27 | First Light receiving device |
| 28 | Second Light receiving device |
| 29 | Projection Shape |

## PREFERRED EMBODIMENTS OF THE INVENTION

Hereafter, embodiments of the present invention will be described with referring to drawings.

(Embodiment 1)

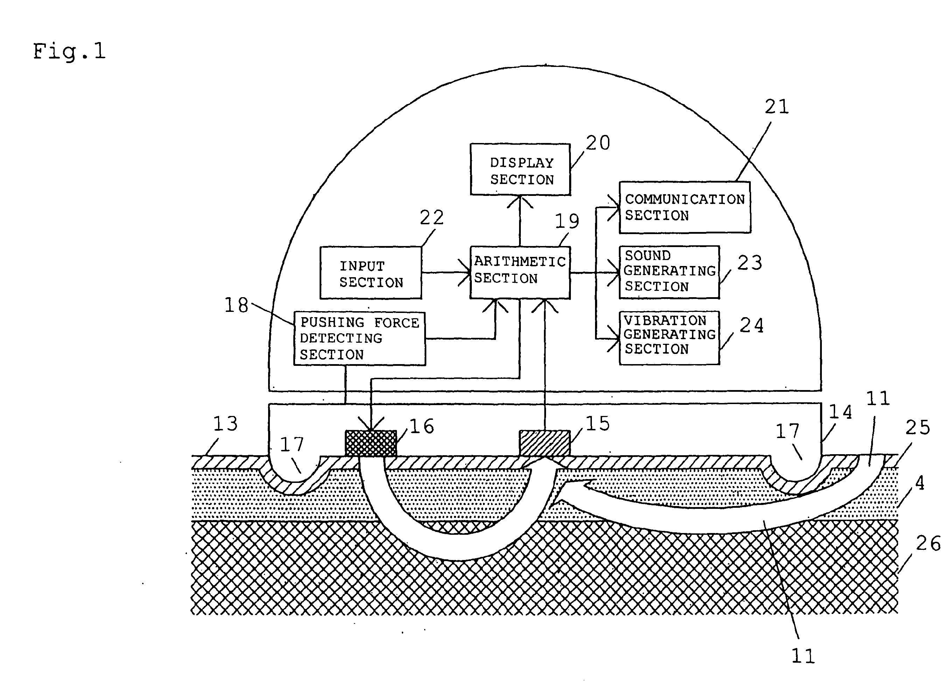

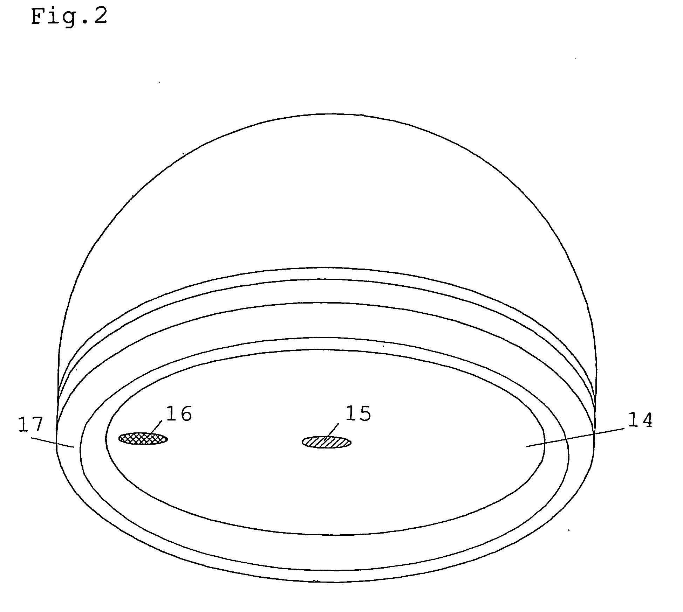

First, the configuration of an optical fat thickness meter of this embodiment will be described, mainly referring to Figures 1 and 2.

In addition, Figure 1 is a schematic sectional view of the optical fat thickness meter of a first embodiment of the present invention. Furthermore, Figure 2 is an outside view of the optical fat thickness meter of the first embodiment of the present invention (in Figure 2, an auxiliary line is entered in a most convex portion of the protruding section 17).

Here, Figure 1 is a sectional view with regard to a plane which includes a photoreception section 15 and a light source section 16 and which is perpendicular to a flat portion of a living body surface 13 where a measuring surface 14 is not pressed.

The measuring surface 14 is a disc-like face with a diameter of about 100 mm adhering to the living body surface 13, and, a photoreception section 15 including a photo diode which is an optical sensor is located in the nearly center thereof.

A light source section 16 including a light emitting diode, which emits near-infrared light at the wavelength of about 850 nm, is located in the distance of 45 mm from the photoreception section 15 on the measuring surface 14.

Here, since Japanese subcutaneous fat thickness exists in a range of, for example, about 0 to 60 mm in many cases, in order to perform subcutaneous fat thickness measurement, it is preferable that distance between the photoreception section 15 and light source section 16 is in a range of about 35 to 50 mm.

Around the measuring surface 14, the protruding section 17 with the height of 5 mm is located circumferentially lest stability should be lost because the device is shaky at the time of use.

The protruding section 17 has a round shape lest a living body should feel pain when touching the living body.

In addition, as for the material of the protruding section 17, it is preferable to have moderate flexibility lest a living body should feel pain when the living body is touched. Furthermore, it is preferable to have low reflectance so as to be able to absorb the near-infrared light at the wavelength of about 850 nm which a light emitting diode which the light source section 16 includes emits. There exists a black ABS resin or the like as a specific example of material of having such flexibility and low reflectance.

A pushing force detecting section 18 is means of detecting a force of pushing the measuring surface 14 on the living body surface 13.

The pushing force detecting section 18 has the configuration of a force gauge which uses a load cell or a strain gauge for a sensor, or the configuration of combining a spring with a pushbutton switch.

An arithmetic section 19 is means of controlling the light source section 16 and calculating partial biological information such as subcutaneous fat thickness from a signal which is obtained from the photoreception section 15.

In addition, the arithmetic section 19 is means of calculating health condition information, including a degree of obesity of a living body and the like, from the information, which is inputted from an input section 22, and the partial biological information.

A display section 20 is means of displaying the partial biological information calculated.

A communication section 21 is means of transmitting and receiving gender information, age information, measurement region information, and the like between with external equipment.

In addition, the communication section 21 is means of transmitting the partial biological information, health condition information, and the like, which are calculated by the arithmetic section 19, to external equipment.

The input section 22 is means of inputting gender, age, measurement region information, and the like.

A sound generating section 23 is means of telling a user about the start to end of measurement with voice.

A vibration generating section 24 is means of telling a user about the start to end of measurement with vibration.

In addition, the protruding section 17 corresponds to a protruding section of the present invention, the light source section 16 corresponds to light source of the present invention, the photoreception section 15 corresponds to photodetector of the present invention, and the arithmetic section 19 corresponds to biological information arithmetic unit of the present invention. Furthermore, the pushing force detecting section 18 corresponds to means including measuring surface pushing force measuring unit and judging unit of the present invention. Moreover, the optical fat thickness meter of this embodiment corresponds to a biological information arithmetic apparatus of the present invention.

Next, the operation of the optical fat thickness meter of this embodiment will be described. In addition, one embodiment of the biological information arithmetic method of the present invention will be also described, with describing the operation of the optical fat thickness meter of this embodiment (this is also the same in the following embodiments).

A user presses the measuring surface 14 against a measurement region.

The pushing force detecting section 18 judges whether a pushing force equal to or beyond a rated value is applied.

Here, the rated value is a value of a force which presses the measuring surface 14 at the time when the fat thickness which was obtained beforehand stops thinning more.

When the pushing force detecting section 18 judges that the pushing force equal to or beyond the rated value is applied, the arithmetic section 19 calculates a signal $V^0$ corresponding to a component, which the disturbance light 11 has propagated, on the basis of the amount of received light in the photoreception section 15.

Next, the light source section 16 emits light.

Then, the arithmetic section 19 calculates a signal V on the basis of the amount of received light in the photoreception section 15 at this time.

The arithmetic section 19 calculates a signal W corresponding to the amount of received light which was emitted by the light source section 16 and has propagated to the photoreception section 15 as follows:

$$(\mathrm{Formula}\ 1)$$

$$W\ =\ V\ -\ V^0$$

Near-infrared light is strongly scattered on the subcutaneous fat 4 in comparison with skin 25 and muscle 26, and there is little absorption.

Hence, the near-infrared light propagates the interior of fat well, and W increases as subcutaneous fat thickness is thick.

The arithmetic section 19 uses such a principle and calculates subcutaneous fat thickness X on the basis of the signal W corresponding to the amount of received light which was emitted by the light source section 16 and has propagated to the photoreception section 15.

The display section 20 displays the subcutaneous fat thickness X calculated.

Up to this point, the configuration and operation of the optical fat thickness meter of this embodiment is described.

In this way, it is possible to suppress the fluctuation of the pushing force every measurement by pushing the measuring surface 14 on the living body surface 13 and starting measurement.

In addition, since the subcutaneous fat 4 is soft in comparison with the skin 25 and muscles 26 when the measuring surface 14 is pressed, the subcutaneous fat which exists directly under the protruding section 17 is crushed locally and becomes thin.

Then, since it becomes difficult that the light propagates directly under the protruding section 17, the disturbance light 11 from the sun or an illumination light decreases, and $V^0$ also decreases.

As a result, an error of measurement of W under the influence of $V^0$ decreases, and hence, the accuracy of measurement of the subcutaneous fat thickness X also improves.

(Embodiment 2)

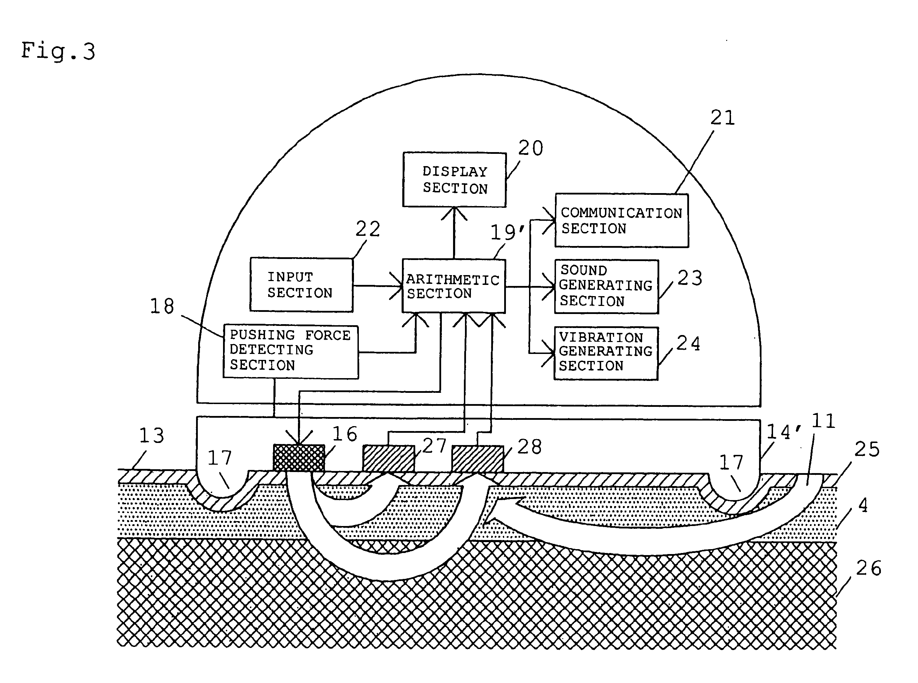

First, the configuration of an optical fat thickness meter of this embodiment will be described, mainly referring to Figures 3 and 4.

In addition, Figure 3 is a schematic sectional view of the optical fat thickness meter of the second embodiment of the present invention. Furthermore, Figure 4 is an outside view of the optical fat thickness meter of the second embodiment of the present invention (in Figure 4, an auxiliary line is drawn in a most convex portion of the protruding section 17).

Here, Figure 3 is a sectional view with regard to a plane which includes a light source 16, a first light receiving device 27, and a second light receiving device 28 and which is perpendicular to a flat portion of the living body surface 13 where the measuring surface 14 is not pressed.

The configuration of the optical fat thickness meter of this embodiment is similar to the configuration of the optical fat thickness meter of the first embodiment mentioned above.

Nevertheless, the optical fat thickness meter of this embodiment is equipped with the first light receiving device 27 and second light receiving device 28 instead of the photoreception section 15 (refer to Figure 1).

The second light receiving device 28 is located nearly in the center of a measuring surface 14', and the first light receiving device 27 is located between the second light receiving device 28 and light source section 16.

In this embodiment, distance between the first light receiving device 27 and light source section 16 is 23 mm, and distance between the second light receiving device 28 and light source section 16 is 45 mm.

Here, as mentioned above, for example, Japanese subcutaneous fat thickness exists in a range of about 0 to 60 mm in many cases, and distance between the light receiving device and light source section where the influence of individual difference of skin appears remarkably is in a range of about 15 to 25 mm. Distance between the light receiving device and light source section which the influence of individual difference of skin, and the influences of subcutaneous fat thickness appear remarkably is in a range of about 35 to 50 mm in many cases. Hence, in order to perform the dispersion correction of the individual difference of skin, it is preferable that distance between the first light receiving device 27 and light source section 16 is in a range of about 15 to 25 mm, and that distance between the second light receiving device 28 and light source section 16 is in a range of about 35 to 50 mm.

In addition, means including the first light receiving device 27 and second light receiving device 28 corresponds to photodetector of the present invention, and arithmetic section 19' corresponds to biological information arithmetic unit of the present invention. Furthermore, the pushing force detecting section 18 corresponds to means including measuring surface pushing force measuring unit and judging unit of the present invention. Moreover, the optical fat thickness meter of this embodiment corresponds to a biological information arithmetic apparatus of the present invention.

Next, the operation of the optical fat thickness meter of this embodiment will be described.

A user presses a measuring surface 14' against a measurement region.

The pushing force detecting section 18 judges whether a pushing force equal to or beyond a rated value is applied.

When the pushing force detecting section 18 judges that the pushing force equal to or beyond the rated value is applied, the arithmetic section 19' calculates a signal $V^0_1{}'$ corresponding to a component, which the disturbance light 11 has propagated, on the basis of the amount of received light in the first light receiving device 27.

Similarly, the arithmetic section 19' calculates a signal $V^0_2{}'$ corresponding to a component, which the disturbance light 11 has propagated, on the basis of the amount of received light in the second light receiving device 28.

Next, the light source section 16 emits light.

Then, the arithmetic section 19' calculates a signal $V_1{}'$ on the basis of the amount of received light in the first light receiving device 27 at this time. The arithmetic section 19' calculates a signal $W_1{}'$ corresponding to the amount of received light which was emitted by the light source section 16 and has propagated to the first light receiving device 27 as follows:

$$(\text{Formula } 2)$$

$$W_1{}' = V_1{}' - V^0_1{}'$$

Similarly, the arithmetic section 19' calculates a signal $V_2{}'$ on the basis of the amount of received light in the second light receiving device 28 at this time. The arithmetic section 19' calculates a signal $W_2{}'$ corresponding to the amount of received light which was emitted by the light source section 16 and has propagated to the second light receiving device 28 as follows:

(Formula 3)

$$W_2' = V_2' - V^0_2'$$

On the basis of the signal $W_1'$ corresponding to the amount of received light which was emitted by the light source section 16 and has propagated to the first light receiving device 27, and the signal $W_2'$ corresponding to the amount of received light which was emitted by the light source section 16 and has propagated to the second light receiving device 28, the arithmetic section 19' performs the dispersion correction of individual difference of the skin 25, and calculates the subcutaneous fat thickness X'.

The display section 20 displays the subcutaneous fat thickness X' calculated.

Up to this point, the configuration and operation of the optical fat thickness meter of this embodiment is described.

In this way, the measurement of the subcutaneous fat thickness where the dispersion of individual difference of color and thickness of the skin 25 is corrected is attained by calculating the subcutaneous fat thickness from a ratio of two signals corresponding to the amount of received light which has propagated through a living body from the light source section 16 and is received by the first light receiving device 27 and second light receiving device 28.

(Embodiment 3)

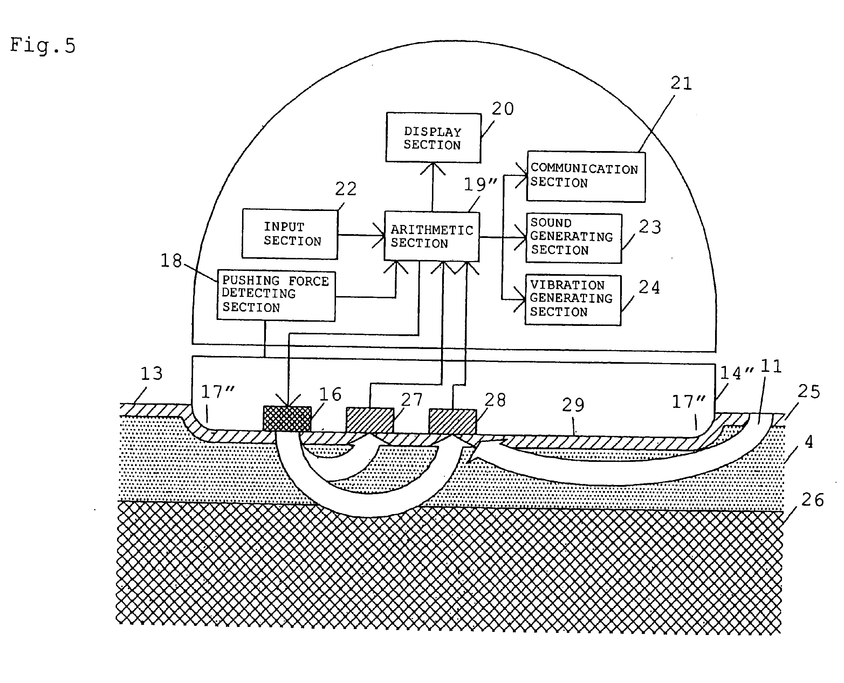

First, the configuration of an optical fat thickness meter of this embodiment will be described, mainly referring to Figures 5, 6, and 7.

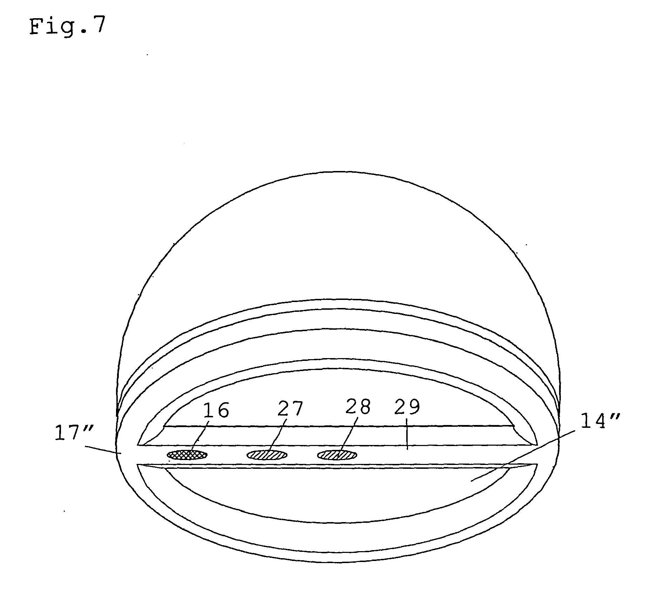

In addition, Figure 5 is a schematic sectional view (part 1) of the optical fat thickness meter of a third embodiment of the present invention. Furthermore, Figure 6 is a schematic sectional view (part 2) of the optical fat thickness meter of the third embodiment of the present invention. Moreover, Figure 7 is an outside view of the optical fat thickness meter of the third embodiment of the present invention.

Here, Figure 5 is a sectional view with regard to a first plane which includes the light source 16, first light receiving device 27, and second light receiving device 28, and which is perpendicular to a flat portion of the living body surface 13 where the measuring surface 14" is not pressed. Figure 6 is a sectional view with regard to a second plane which includes the second light receiving device 28, and which is perpendicular to a flat portion of the living body surface 13, where the measuring surface 14" is not pressed, and the first plane.

The configuration of the optical fat thickness meter of this embodiment is similar to the configuration of the optical fat thickness meter of the second embodiment mentioned above.

Nevertheless, the optical fat thickness meter of this embodiment is equipped with a long and slender protruding shape 29 with 5 mm high x 5 mm wide which is provided in the center of the measuring surface 14" having the light source section 16, first light receiving device 27, and second light receiving device 28, so as to bridge to a protruding section 17".

The second light receiving device is located nearly in the center of the protruding shape 29, and the first light receiving device 27 is located between the second light receiving device 28 and light source section 16.

In this embodiment, distance between the first light receiving device 27 and light source section 16 is 23 mm, and distance between the second light receiving device 28 and light source section 16 is 45 mm.

Hence, because of the reason described above, in order to perform the dispersion correction of the individual difference of skin, it is preferable that distance between the first light receiving device 27 and light source section 16 is in a range of about 15 to 25 mm, and that distance between the second light receiving device 28 and light source section 16 is in a range of about 35 to 50 mm.

In addition, the protruding section 17" corresponds to a protruding section of the present invention, means including the first light receiving device 27 and second light receiving device 28 corresponds to photodetector of the present invention, and arithmetic section 19" corresponds to biological information arithmetic unit of the present invention. Furthermore, the projection shape 29 corresponds to a convex section of the present invention. Moreover, the pushing force detecting section 18 corresponds to convex section pushing force measuring unit of the present invention. In addition, the optical fat thickness meter of this embodiment corresponds to a biological information arithmetic apparatus of the present invention.

Next, the operation of the optical fat thickness meter of this embodiment will be described.

A user presses the measuring surface 14" against a measurement region.

The pushing force detecting section 18 judges whether a pushing force equal to or beyond a rated value is applied.

When the pushing force detecting section 18 judges that the pushing force equal to or beyond the rated value is applied, the arithmetic section 19" calculates a signal $V^0_1$" corresponding to a component, which the disturbance light 11 has propagated, on the basis of the amount of received light in the first light receiving device 27.

Similarly, the arithmetic section 19" calculates a signal $V^0_2$" corresponding to a component, which the disturbance light 11 has propagated, on the basis of the amount of received light in the second light receiving device 28.

Next, the light source section 16 emits light.

Then, the arithmetic section 19" calculates a

signal $V_1''$ on the basis of the amount of received light in the first light receiving device 27 at this time. The arithmetic section 19" calculates a signal $W_1''$ corresponding to the amount of received light which was emitted by the light source section 16 and has propagated to the first light receiving device 27 as follows:

(Formula 4)

$$W_1'' = V_1'' - V^0_1''$$

Similarly, the arithmetic section 19" calculates a signal $V_2''$ on the basis of the amount of received light in the second light receiving device 28 at this time. The arithmetic section 19" calculates a signal $W_2''$ corresponding to the amount of received light which was emitted by the light source section 16 and has propagated to the second light receiving device 28 as follows:

(Formula 5)

$$W_2'' = V_2'' - V^0_2''$$

On the basis of the signal $W_1''$ corresponding to the amount of received light which was emitted by the light source section 16 and has propagated to the first light receiving device, and the signal $W_2''$ corresponding to the amount of received light which was emitted by the light source section 16 and has propagated to the second light receiving device, the arithmetic section 19" performs the dispersion correction of individual difference of the skin 25, and calculates the subcutaneous fat thickness $X''$.

The display section 20 displays the subcutaneous fat thickness $X''$ calculated.

Up to this point, the configuration and operation of the optical fat thickness meter of this embodiment is described.

In this way, similarly to the case in the second embodiment, the measurement of the subcutaneous fat thickness where the dispersion of individual difference of color and thickness of the skin 25 is corrected is attained by calculating the subcutaneous fat thickness from a ratio of two signals corresponding to the amount of received light which has propagated through a living body from the light source section 16 and is received by the first light receiving device 27 and second light receiving device 28.

In addition, since the arithmetic section 19" converts into original subcutaneous fat thickness the subcutaneous fat thickness, compressed and measured by the projection shape 29, in consideration of the deformation of the living body surface 13 owing to the pushing force detected by the pushing force detecting section 18, it becomes possible also to measure thicker subcutaneous fat thickness. Furthermore, such conversion may be performed using a conversion formula which was deduced beforehand by investigating the relationship between the subcutaneous fat thickness, compressed by a pushing force, and the original subcutaneous fat thickness, which is not compressed, by ultrasonic diagnostic equipment or the like.

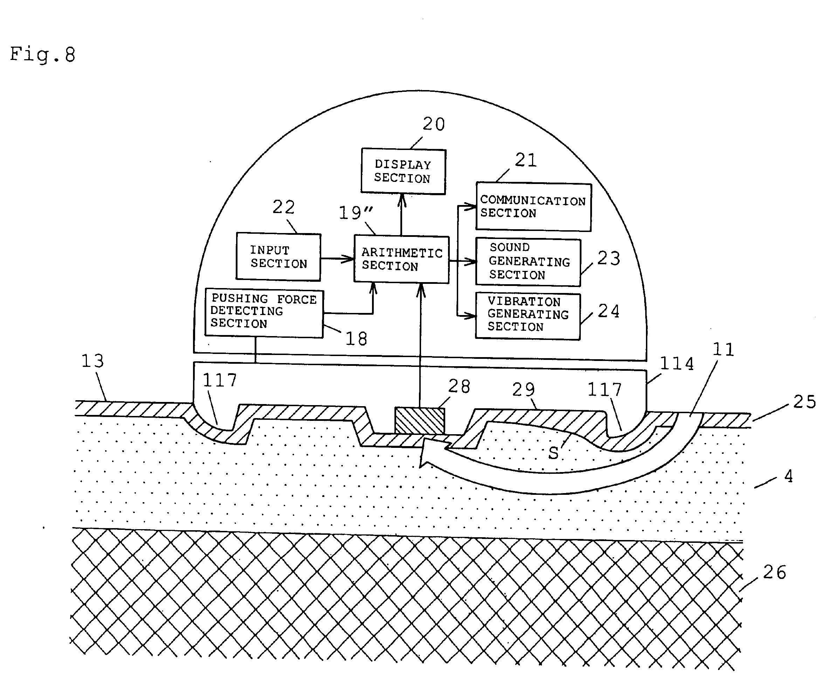

(A) In addition, a protruding section of the present invention may be a protruding section 117, which is provided along the outer periphery of the measuring surface 114, having a curved surface part S, whose curved condition varies gently toward the projection shape 29, in its interior as shown in Figure 8 which is a schematic partially-sectional view of an optical fat thickness meter with a protruding section 117 in an embodiment of the present invention.

When the protruding section 117 is pressed against the skin 25 since the curved condition of the curved surface part S varies gently, the sense of discomfort which a user feels is decreased, and dust and the like hardly collect on the curved surface part S.

Of course, since the curved surface part S is provided in the inside of the protruding section 117 instead of the outside of the protruding section 117, photoreception of disturbance light from the sun or illumination light is suppressed similarly to the cases in the embodiments mentioned above.

In addition, the protruding section 117 corresponds to a convex section of the present invention.

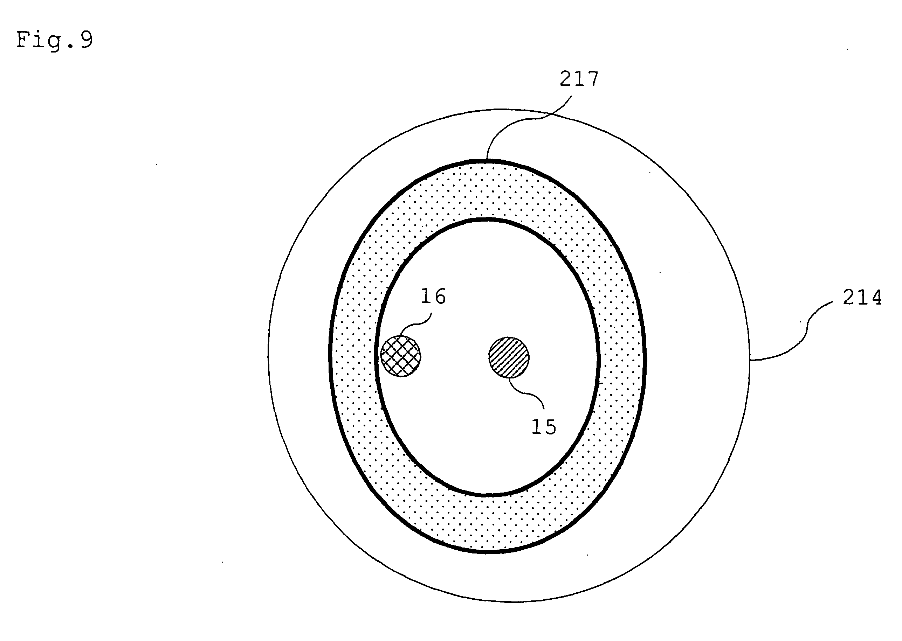

(B) Furthermore, as shown in Figure 9 which is a schematic bottom view of an optical fat thickness meter with a protruding section 217 according to an embodiment of the present invention, the protruding section of the present invention may be provided in some extent of distance from the outer periphery of a measuring surface 214, not along the outer periphery of the measuring surface 214.

Since the protruding section 217 is provided so as to surround the light source section 16 and photoreception section 15, the photoreception of disturbance light from the sun or illumination light is suppressed similarly to the cases in the embodiments mentioned above. Hence, it is possible to enhance the accuracy of measurement of partial subcutaneous fat thickness and the like further.

In addition, the protruding section 217 corresponds to a convex section of the present invention.

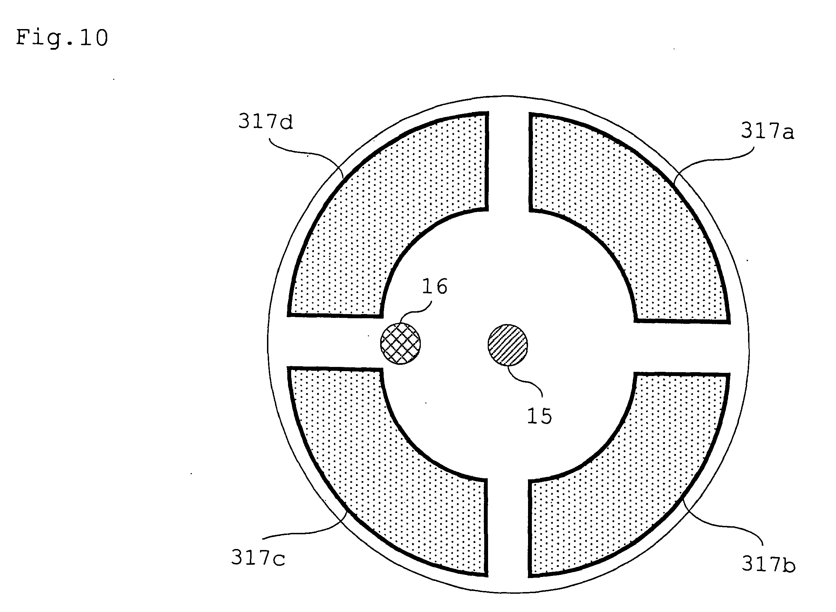

(C) Furthermore, as shown in Figure 10 which is a schematic bottom view of an optical fat thickness meter with protruding sections 317a, 317b, 317c, and 317d according to an embodiment of the present invention, the protruding section of the present invention may be provided intermittently, not continuously.

Since the protruding sections 317a, 317b, 317c, and 317d are provided so as to surround the light source section 16 and photoreception section 15 substantially, the photoreception of disturbance light from the sun or illumination light is suppressed similarly to the cases in the embodiments mentioned above. Hence, it is possible further to enhance the accuracy of measurement of partial subcutaneous fat thickness and the like.

Of course, although it becomes difficult to suppress the photoreception of disturbance light when the size of the intermission between protruding sections is too large, when the size of such intermission is suitable, it is possible to decrease the sense of discomfort, which a user feels, while suppressing the photoreception of disturbance light.

In addition, the protruding sections 317a, 317b, 317c, and 317d correspond to a convex section of the present invention.

The embodiments of the present invention are described above in detail.

In this way, the optical biological information measuring apparatus according to the present invention can press, for example, against a living body a measuring surface which has a protruding section around to compress a subcutaneous fat layer directly under the protruding section, and can decrease disturbance light.

Therefore, the optical biological information measuring apparatus according to the present invention is useful as an optical biological information measuring apparatus which measures partial biological information such as partial fat thickness.

In addition, the optical biological information measuring apparatus according to the present invention is applicable also to applications such as an optical type partial fabric oximeter.

In addition, the program of the present invention is a program for making a computer execute the operation of all or a part of steps of the biological information arithmetic methods of the present invention mentioned above, and is a program which operates with collaborating with a computer.

Furthermore, the recording medium of the present invention is a recording medium which holds a program for executing the operation of all or a part of steps of the biological information arithmetic methods of the present invention mentioned above by computer, the program in which can be read by a computer, and executes the above-mentioned operation with collaborating with the above-mentioned computer.

Moreover, the above-mentioned "a part of steps" of the present invention means one or some steps of a plurality of those steps.

In addition, the above-mentioned "operation of a step" of the present invention means the operation of all or a part of the above-mentioned step.

Furthermore, one utilizing form of the program of the present invention may be an aspect which is recorded on a recording medium which can be read by a computer, and operates with collaborating with the computer.

Moreover, another utilizing form of the program of the present invention may be an aspect which is transmitted inside a transmission medium, is read by a computer, and operates with collaborating with the computer.

In addition, as recording media, ROM and the like are included, and as transmission media, transmission media such as the Internet, light, electric waves, acoustic waves, and the like are included.

Furthermore, the computer of the present invention mentioned above may be not only pure hardware such as a CPU, but also firmware, OS, and further, what includes a peripheral device.

Moreover, as described above, the configuration of the present invention may be achieved in software or hardware.

The biological information arithmetic apparatus, biological information calculation method, program, and recording medium of the present invention can further enhance the accuracy of measurement of such as partial subcutaneous fat thickness to be useful.

**Claims**

1. A biological information arithmetic apparatus, comprising:

   a light source emitting light to a living body, which is provided on a measuring surface;
   photodetector receiving the light having passed through the living body, which is provided on the measuring surface;
   a protruding section which is provided so as to substantially surround the light source and the photodetector; and
   a biological information arithmetic unit of calculating information with regard to the living body on the basis of an amount of the received light.

2. The biological information arithmetic apparatus according to claim 1,
   wherein the photodetector receives the light also in a state where the light is not emitted while receiving the light in a state where the light is emitted, and the biological information arithmetic unit calculates the information with regard to the living body on the basis of the amount of the light received in the state where the light is not emitted, and the amount of the light received in the state where the light is emitted.

3. The biological information arithmetic apparatus according to claim 1, wherein the protruding section is continuously provided along an outer periphery of the measuring surface.

4. The biological information arithmetic apparatus according to claim 3,

wherein the outer periphery of the measuring surface is substantially a circumference, and the photodetector is located in a substantial center of the circumference.

5. The biological information arithmetic apparatus according to claim 1, further comprising a convex section provided so as to bridge in the protruding section, wherein the light source is located in the convex section, and the photodetector is located in the convex section.

6. The biological information arithmetic apparatus according to claim 5, wherein the outer periphery of the measuring surface is substantially a circumference, and the convex section is provided so as to cross a substantial center of the circumference.

7. The biological information arithmetic apparatus according to claim 5, further comprising a convex section pushing force measuring unit of measuring a pushing force by which the convex section is pushed on a surface of the living body, wherein the biological information arithmetic unit calculates the information with regard to the living body in consideration of a deformation of the surface of the living body which is caused by the measured pushing force.

8. The biological information arithmetic apparatus according to claim 1, wherein the photodetector has a first light receiving device that receives the light, and a second light receiving device that receives the light, and the biological information arithmetic unit calculates the information with regard to the living body on the basis of an amount of the light received by the first light receiving device, and an amount of the light received by the second light receiving device.

9. The biological information arithmetic apparatus according to claim 8, wherein the light source has a light emitting device which emits the light, a distance between the light emitting device and the first light receiving device is substantially from 15 mm or more to 25 mm or less, and a distance between the light emitting device and the second light receiving device is substantially from 35 mm or more to 50mm or less.

10. The biological information arithmetic apparatus according to claim 1, further comprising:

    a measuring surface pushing force measuring unit of measuring a pushing force by which the measuring surface is pushed on a surface of the living body; and a judging unit of judging whether a magnitude of the measured pushing force is over a predetermined value, wherein the biological information arithmetic unit calculates the information with regard to the living body if the magnitude of the measured pushing force is judged to be over the predetermined value.

11. A biological information arithmetic method, comprising the steps of:

    emitting light using a light source which is provided on a measuring surface so as to be substantially enclosed by a protruding section and which emits light to a living body; performing photoreception using a photodetector which is provided on the measuring surface so as to be substantially enclosed by the protruding section and which receives light having passed through the living body; and performing an arithmetical operation using a biological information calculator which calculates information with regard to the living body on the basis of an amount of the received light.

12. A program for making a computer execute the step of performing an arithmetical operation using a biological information calculator which calculates information with regard to the living body on the basis of an amount of the received light, in the biological information arithmetic method according to claim 11.

13. A recording medium recording the program according to claim 12, which can be processed by a computer.

Fig.1

EP 1 627 599 A2

Fig.2

Fig.3

DISPLAY
SECTION 20

COMMUNICATION
SECTION 21

INPUT
SECTION 22

ARITHMETIC
SECTION 19'

SOUND
GENERATING
SECTION 23

PUSHING FORCE
DETECTING
SECTION 18

VIBRATION
GENERATING
SECTION 24

13    17    16    27    28    17    14'    11    25    4    26

Fig.4

Fig.5

Fig.6

EP 1 627 599 A2

Fig.7

Fig.8

EP 1 627 599 A2

Fig.9

EP 1 627 599 A2

EP 1 627 599 A2

Fig.10

317d

317a

16

15

317c

317b

Fig.11    PRIOR ART

10

MEASUREMENT REGION
SELECTION INPUT SECTION

ARM    LEG    BELLY

1

CPU

MEASUREMENT
VALUE
DISPLAY
SECTION

2

DRIVE

9

AMP

8

ANALOG SWITCH

3    5    6    7

4    11

21